# DEMANDE DE BREVET EUROPEEN

(11) **EP 1 457 198 A1**
(43) Date de publication de la demande: **15.09.2004**
(21) Numéro de dépôt: 03292455.7
(22) Date de dépôt: 03.10.2003
(51) Int. Cl.: A61K 7/13

(54) **Composition tinctoriale comprenant au moins une base d'oxydation diaminopyrazole et au moins un coupleur 6-alcoxy 2,3-diaminopyridine**

(30) Priorité: 04.10.2002 FR 0212353
(71) Demandeur: L'OREAL, 75008 Paris (FR)
(72) Inventeur: Kravtchenko, Sylvain, 92600 Asnieres (FR); Lagrange, Alain, 77700 Coupvray (FR)
(74) Mandataire: Wattremez, Catherine

(57) **Abrégé**

L'invention a pour objet une composition tinctoriale comprenant au moins une base d'oxydation du type diaminopyrazole et au moins un coupleur du type 6-alcoxy 2,3-diaminopyridine.

L'invention a aussi pour objet l'utilisation dé cette composition pour la teinture des fibres kératiniques ainsi que le procédé de teinture mettant en oeuvre cette composition.

La composition de la présente invention permet en particulier d'obtenir des nuances foncées telles que des nuances grises à noires.

## Description

L'invention a pour objet une composition tinctoriale comprenant au moins une base d'oxydation du type diaminopyrazole et au moins un coupleur du type 6-alcoxy 2,3-diaminopyridine. L'invention a aussi pour objet l'utilisation de cette composition pour la teinture des fibres kératiniques ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine, ou entre des cheveux différemment sensibilisés au sein d'une même chevelure.

Il est déjà connu d'utiliser des compositions tinctoriales comprenant en tant que base d'oxydation des composés diaminopyrazole pour la teinture de fibres kératiniques.

Par exemple, le brevet US 5 061 289 décrit des compositions pour la teinture d'oxydation des fibres kératiniques comprenant une base d'oxydation du type diaminopyrazole. Ces bases d'oxydation permettent d'obtenir des nuances variées, en particulier dans le domaine des rouges. Cependant, elles ne permettent pas d'obtenir des nuances foncées, en particulier des nuances grises à noires.

La demande de brevet DE 19643059 décrit des compositions tinctoriales associant des bases d'oxydation diaminopyrazole avec des coupleurs particuliers du type méta-aminophénol et métaphénylènediamine. Ces associations particulières permettent d'obtenir des nuances variées dans le domaine des rouges.

Par ailleurs, il est connu d'utiliser dans le domaine de la coloration des fibres kératiniques des coupleurs pyridines. Le document US 4 784 667 décrit en particulier des coupleurs 2,3-diamino 6-méthoxypyridine. Ces coupleurs lorsqu'ils sont utilisés avec des bases benzéniques du type diamine aromatique, 2-aminophenol ou 4-aminophénol permettent d'obtenir des couleurs intenses et stables dans les nuances bleues.

Le but de la présente invention est de développer de nouvelles compositions tinctoriales permettant d'obtenir une coloration des fibres kératiniques dans des nuances foncées, en particulier des nuances variant du gris au noir, neutres qui sont peu sélectives et qui présentent une bonne ténacité vis à vis des différentes agressions extérieures que peuvent subir les cheveux.

Ce but est atteint avec la présente invention qui a pour objet une composition tinctoriale comprenant, dans un milieu de teinture approprié :
- au moins un coupleur pyridinique de formule (I) : 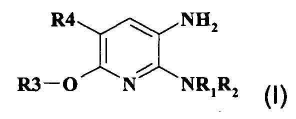 dans laquelle
   - R₁ et R₄ représentent un atome d'hydrogène ; un radical alkyle linéaire ou ramifié de 1 à 6 atomes de carbone, éventuellement substitué par un ou plusieurs atomes d'halogène, un ou plusieurs radicaux hydroxy, amino, mono-di- ou tri- alkylamino, aryle, alcoxyC₁-C₃ ou hétérocyclique ; un radical arylC₆-C₁₈, éventuellement substitué par un ou plusieurs radicaux amino, hydroxy, alcoxy(C₁-C₃) ou alkyl(C₁-C₆) ; un radical carboxyalkyl(C₁-C₆) ou un radical alkylthio(C₁-C₆) (RS-) ; un radical alcényle en C₂-C₆ ;
   - R₂ représente un radical alkyle linéaire ou ramifié de 2 à 6 atomes de carbone, éventuellement substitué par un ou plusieurs atomes d'halogène, un ou plusieurs radicaux hydroxy, amino, di ou trialkylaminoaryle, alcoxyC₁-C₃ ou hétérocyclique ; un radical aryl C₆-C₁₈, éventuellement substitué par un ou plusieurs radicaux amino, hydroxy, alcoxy(C₁-C₃) ou alkyl(C₁-C₆) ; un radical carboxyalkyl(C₁-C₆) ou un radical alkylthio(C₁-C₆) ; un radical alcényle en C₂-C₆ ; ou
   - R₁ et R₂, ensemble, forment avec l'atome d'azote auquel ils sont rattachés un hétérocycle ayant de 3 à 18 chaînons, pouvant contenir un ou plusieurs hétéroatomes supplémentaires choisis parmi un atome d'oxygène, d'azote ou de soufre, cet hétérocycle pouvant être substitué par un ou plusieurs radicaux alkyle en C₁-C₆, amino, acylamino C2-C6 ou hydroxyalkyle(C₁-C₄), le cycle ne comportant pas de liaison peroxyde, ni de radicaux diazo ou nitroso ;
   - R₃ représente un radical alkyle de 1 à 6 atomes de carbone, éventuellement substitué par un ou plusieurs atomes d'halogène, un ou plusieurs radicaux hydroxy, amino, aryle, alcoxyC₁-C₃ ; un radical aryl C₆-C₁₈, éventuellement substitué par un ou plusieurs radicaux amino, hydroxy, alcoxy(C₁-C₃), alkyl(C₁-C₆) ; un radical carboxyalkyl(C₁-C₆) ou un radical alkylthio(C₁-C₆) ;
- au moins une base d'oxydation hétérocyclique à motifs pyrazoliques non condensés ;
- étant entendu que lorsque le coupleur pyridinique de formule (I) est choisi parmi la 2-(β-hydroxyéthylamino) 6-méthoxy 3-amino pyridine et la 3-amino 6-méthoxy 2-(méthylamino) pyridine, le pH de la composition prête à l'emploi est inférieur à 6,5.

L'invention a aussi pour objet des procédés de teinture des fibres kératiniques à partir de cette composition.

Un autre objet de l'invention est l'utilisation de la composition de la présente invention pour la teinture des fibres kératiniques, en particulier les fibres kératiniques humaines telles que les cheveux.

La composition de la présente invention permet en particulier d'obtenir une coloration de fibres kératiniques dans des nuances foncées grises à noires, tenaces, et stables, en particulier aux shampoings. Les colorations obtenues à partir des compositions de l'invention présentent une meilleure intensité tout en réduisant le virage des nuances lorsqu'elles subissent une action extérieure (lumière, sueur, ou des traitements cosmétiques tels que les shampooing ou la permanente).

Dans le cadre de la présente invention, on entend par alkyle, des radicaux linéaires ou ramifiés, par exemple méthyle, éthyle, n-propyle, iso-propyle, butyle, etc.

Un radical aryle en C₆-C₁₈ est un radical par exemple choisi parmi un radical phényle, un radical benzyle, un radical naphtyle.

Selon un mode de réalisation particulier, le coupleur pyridinique est choisi parmi les composés de formule (I) à l'exception de la 2-(β-hydroxyéthylamino) 6-méthoxy 3-amino pyridine et la 3-amino 6-méthoxy 2-(méthylamino) pyridine.

Selon un mode de réalisation particulier, R₂ représente un radical mono ou polyhydroxyalkyle en C₁-C₄.

Selon un mode de réalisation différent, R₁ et R₂ forment ensemble avec l'atome d'azote auquel ils sont attachés un cycle pyrrolidine, pipéridine, homopipéridine, imidazoline, pyrazolidine, piperazine.

R₃ représente de préférence un radical alkyle en C1-C6 ou trifluoroalkyle.

R₄ est de préférence un atome d'hydrogène.

La base d'oxydation hétérocyclique à motifs pyrazoliques non condensés est par exemple une base d'oxydation diaminopyrazole de formule (III) 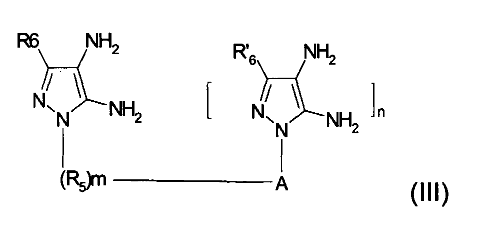 dans laquelle
- R₅, R₆ et R'₆ représentent indépendamment l'un de l'autre un atome d'hydrogène ; une chaîne alkyle de 1 à 6 atomes de carbone, éventuellement substituée par un ou plusieurs radicaux choisis parmi les radicaux hydroxy, amino, mono-, di- ou tri-alkylC1-C4amino, alcoxy(C₁-C₄), aryl(C₆-C₁₈) ou un ou plusieurs atomes d'halogène ; un radical aryle en C₆-C₁₈, éventuellement substitué par un ou plusieurs radicaux choisis parmi un radical amino, hydroxy, alcoxy(C₁-C₃), alkyle(C₁-C₆) ; un radical carboxyalkyl(C₁-C₆) ou alkylthio(C₁-C₆),
- n désigne 0 ou 1 ;
- m désigne 0 ou 1 à la condition que si n = 1 alors m = 0 et si n = 0 alors m= 1 ;
- A désigne une chaîne hydrocarbonée en C₁-C₂₀, linéaire ou ramifiée, cyclique ou non, saturée ou insaturée, hétérocyclique ou non, éventuellement interrompue par un ou plusieurs hétéroatomes, et éventuellement substituée par un radical hydroxy ou amino, un atome d'halogène.

R₆ est de préférence un atome d'hydrogène ou un radical alkyle en C1-C4.

A titre d'exemple, lorsque n est égal à 0, les bases d'oxydation diaminopyrazole utiles dans la composition de l'invention peuvent être le 4,5-diamino 1-méthyl pyrazole, le 4,5-diamino 1-(β-hydroxyéthyl) pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 1-benzyl 4,5-diamino 3-méthyl pyrazole, le 4,5-diamino 3-tert-butyl 1-méthyl pyrazole, 1e.4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 4,5-diamino-1-méthoxyéthylpyrazole, le 4,5-diamino 1-aminoéthylpyrazole, le 4,5-diamino 1-triméthylaminopropylpyrazole, et leurs sels d'addition avec un acide.

Lorsque n est égal à 1, la base d'oxydation hétérocyclique à motifs pyrazoliques non condensés est de préférence un dimère de diaminopyrazole choisi parmi le 1,2 bis-[4,5-diamino-pyrazol-1-yl)éthane, le 1,4-bis-[4,5-diamino-pyrazol-1-yl méthyl]benzène, le 1,3-bis-(4,5-diamino-pyrazol-1-yl-méthyl)benzène, le 1,3-bis-(4,5-diamino-pyrazol-1-yl)propane.

De préférence, la base d'oxydation est choisie parmi le 4,5-diamino-1-β-hydroxyéthylpyrazole, le 4,5-diamino-1-éthyl 3-méthyl pyrazole et le 4,5-diamino 1-méthoxyéthylpyrazole.

Les dérivés de diaminopyrazole utiles pour la présente invention peuvent être préparés selon des méthodes connues et décrites dans la littérature. On pourra se reporter à titre d'exemples aux références suivantes: US 5061289, US 5 380 340, EP 618902, US 5 766 576, EP 871 426, EP 1236460..

A titre d'exemple, les coupleurs pyridiniques utiles dans la présente invention sont choisis parmi : 6-méthoxy 3-amino 2-phénylaminopyridine, 6-méthoxy 3-amino 2-(4'-hydroxyphényl)pyridine, 6-méthoxy 3-amino 2-(2'-méthoxyphényl)aminopyridine, 6-méthoxy 3-amino 2-(2'-hydroxyphényl)aminopyridine, 6-méthoxy 3-amino 2-diéthylaminopyridine, 6-méthoxy 3-amino 2-diméthylaminopyridine, 6 méthoxy 3-amino 2-(méthyl, 2'-hydroxyéthyl)aminopyridine, 6-méthoxy 3-amino 2-(n-butyl, 2'hydroxyéthyl)pyridine, 6-méthoxy 3-amino 2bis-(2'hydroxyéthyl)aminopyridine, 6-méthoxy 3-amino 2-(2'3'dihydroxypropyl)aminopyridine, 6-méthoxy 3-amino 2-(1',1'-diméthyl 2'-hydroxyéthyl)aminopyridine, 6-méthoxy 3-amino 2-(1'-hydroxyméthyl 2'-hydroxyéthyl)aminopyridine, 6-méthoxy 3-amino 2-(1'-méthyl 2'-hydroxyéthyl)aminopyridine, 6-méthoxy 3-amino 2-(2'-hydroxypropyl)aminopyridine, 6-méthoxy 3-amino 2-(3'-diméthylaminopropyl)aminopyridine, 6-méthoxy 3-amino 2bis-(méthoxyéthyl)aminopyridine, 6-méthoxy 3-amino 2bis-(2'-propényl)aminopyridine, 6-méthoxy 3-amino 2-pyrrolidinylpyridine, 6-méthoxy 3-amino 2-(3'acétamidopyrrolidinyl)pyridine, 6-méthoxy 3-amino 2-(2'5'diméthylpyrrolidinyl)pyridine, 6-méthoxy 3-amino 2-(2'-diméthylaminoéthyl)aminopyridine, 6-méthoxy 3-amino 2-morpholinopyridine, 6-méthoxy 3-amino 2-(2'-méthylpyrrolidinyl)pyridine, 6-méthoxy 3-amino 2-pipérazinylpyridine, 6-méthoxy 3-amino 2-pyridinylpyridine, 6-méthoxy 3-amino 2-pyrrolidinylpyridine, 6-méthoxy 3-amino 2-(2'-méthylpyridinyl)pyridine, 6-méthoxy 3-amino 2-(2'-hydroxyéthylpyridinyl)pyridine, 6-méthoxy 3-amino 2-(2'-pyrrolidinyléthyl)aminopyridine, 6-méthoxy 3-amino 2-(3'imidazolinylpropyl)aminopyridine, 6-méthoxy 3-amino 2-[3'-(3"-méthylimidazolium)propyl)aminopyridine, 6-(2'-trifluoroéthoxy) 5-trifluorométhyl 2,3-diamino pyridine, 6-phénoxy 5-trifluorométhyl 2,3-diaminopyridine.

De préférence, les coupleurs pyridiniques utiles sont choisis parmi les composés 6-méthoxy 3-amino 2-(2'3'dihydroxypropyl)aminopyridine, 6-méthoxy 3-amino 2-(1'-méthyl 2'-hydroxyéthyl)aminopyridine, 6-méthoxy 3-amino 2-pyrrolidinylpyridine, 6-méthoxy 3-amino 2-(2'-méthylpyrrolidinyl)pyridine, 6-méthoxy 3-amino 2-(2'-méthylpyridinyl)pyridine, 6-méthoxy 3-amino 2-(2'-hydroxyéthylpyridinyl)pyridine.

Ces coupleurs peuvent être peuvent être préparés selon des méthodes connues et décrites dans la littérature. On pourra se reporter à titre d'exemples au brevet DE 3233540.

Une composition tinctoriale pour la teinture des fibres kératiniques peut en outre comprendre une ou plusieurs bases d'oxydation classiques dans le domaine de la coloration. A titre d'exemple, on peut citer les para-phénylènediamines, les bis-phénylalkylènediamines, les para-aminophénols, les ortho-aminophénols, les bases hétérocycliques autres que celles décrites précédemment et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 2-chloro 4-aminophénol, le 2,6-dichloro 4-amino phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques autres que ceux utiles dans la composition de la présente invention.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés, le 3,4-diamino pyrazole, le 4-amino 1,3-diméthyl 5-hydrazino pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

La composition selon l'invention peut contenir un ou plusieurs coupleurs conventionnellement utilisés pour la teinture de fibres kératiniques autres que le coupleur pyridinique utile dans la présente invention. Parmi ces coupleurs, on peut notamment citer les méta-phénylènediamines, les méta-aminophénols, les méta-diphénols, les coupleurs naphtaléniques, les coupleurs hétérocycliques et leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 4 et 11 environ. Selon un mode de réalisation particulièrement préféré, le pH est compris entre 5 et 8.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 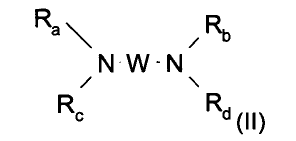 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Selon un premier mode de réalisation, le procédé de teinture des fibres kératiniques de la présente invention est un procédé dans lequel on applique sur les fibres la composition selon la présente invention telle que définie précédemment, en présence d'un agent oxydant pendant un temps suffisant pour développer la couleur désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. Le pH de la composition prête à l'emploi est de préférence inférieur à 9, et plus préférentiellement inférieur ou égal à 7.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré. L'oxygène de l'air peut aussi être utilisé comme agent oxydant.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante prête à l'emploi appliquée sur les fibres kératiniques est inférieur à 9, de préférence inférieur ou égal à 7, généralement compris entre 5 et 7. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un second mode de réalisation du procédé de l'invention consiste à appliquer la composition de l'invention sur les cheveux et de laisser agir l'oxygène de l'air pendant un temps suffisant pour atteindre la coloration souhaitée.

L'invention a aussi pour objet un kit pour la coloration des cheveux comprenant d'une part une composition tinctoriale telle que défini précédemment et d'autre part une composition contenant un agent oxydant, ainsi qu'un dispositif à plusieurs compartiments dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### EXEMPLE DE TEINTURE

On a préparé la composition de teinture, conforme à l'invention, suivante :

| | |
|---|---|
| 4,5-diamino-3-méthyl-1-éthyl -pyrazole | 0,62 g (3.10⁻³mol%) |
| 3-amino-6-methoxy-2-pyrrolidinyl-pyridine dichlorhydrate | 0,94 g (3.10⁻³mol%) |
| Alcool oléique polyglycérolé à 4 moles de glycérol, à 78 % de matières actives (M.A.) | 5,7 g M.A. |
| Alcool oléique polyglycérolé à 2 moles de glycérol | 4,0 g |
| Acide oléique | 3,0 g |
| Amine oléique à 2 moles d'oxyde d'éthylène, vendue sous la dénomination Ethomeen 012 par la Société AKZO | 7,0 g |
| Laurylamino succinamate de diéthylaminopropyle, sel de sodium, à 55 % de M.A | 3,0 g M.A |
| Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35 % de M.A. | 0,46 g M.A. |
| - Acétate d'ammonium | 0,8 g |
| - Antioxydant, séquestrant, | q.s. |
| - Parfum, conservateur, | q.s. |
| - Eau déminéralisée q.s.p. | 100,0 g |

Au moment de l'emploi, on a mélangé cette composition poids pour poids avec de l'eau oxygénée titrant 9 volumes (2,7% en poids), de pH 3. Le pH du mélange est de 7. On applique ce mélange sur des cheveux gris naturels à 90 % de blancs et sur des cheveux gris naturels à 90 % de blancs permanentés pendant 30 minutes. Après rinçage, lavage au shampooing, rinçage et séchage, les cheveux sont teints.

Chaque mèche est évaluée dans le système L*a*b*, au moyen d'un spectrocolorimètre CM 2002 MINOLTA ®, (Illuminant D65).

Dans le système L* a* b*, les trois paramètres désignent respectivement l'intensité (L*), la nuance (a*) et la saturation (b*). Selon ce système, plus la valeur de L est élevée, plus la couleur est claire ou peu intense. Inversement, plus la valeur de L est faible, plus la couleur est foncée ou très intense. a* et b* indiquent deux axes de couleurs, a* indique l'axe de couleur vert/rouge et b* l'axe de couleur bleu/jaune.

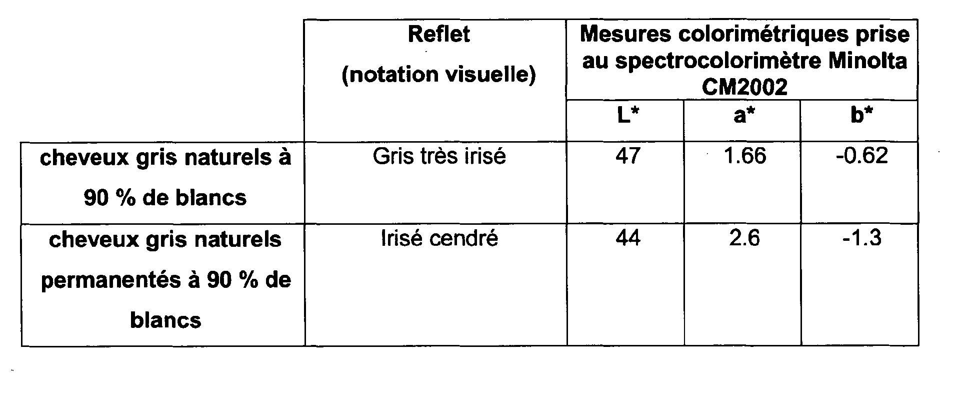

## Revendications

1. Composition tinctoriale comprenant, dans un milieu de teinture approprié,
• au moins un coupleur pyridinique de formule (I) : 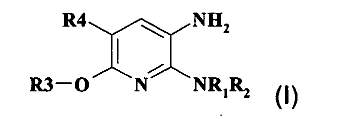 dans laquelle
- R₁ et R₄ représentent un atome d'hydrogène ; un radical alkyle linéaire ou ramifié de 1 à 6 atomes de carbone, éventuellement substitué par un ou plusieurs atomes d'halogène, un ou plusieurs radicaux hydroxy, amino, mono-di- ou tri-alkylamino, aryle, alcoxyC₁-C₃ ou hétérocyclique; un radical arylC₆-C₁₈, éventuellement substitué par un ou plusieurs radicaux amino, hydroxy, alcoxy(C₁-C₃) ou alkyl(C₁-C₆) ; un radical carboxyalkyl(C₁-C₆) ou un radical alkylthio(C₁-C₆) ; un radical alcényle en C2-C6 ;
- R₂ représente un radical alkyle linéaire ou ramifié de 2 à 6 atomes de carbone, éventuellement substitué par un ou plusieurs atomes d'halogène, un ou plusieurs radicaux hydroxy, amino, di ou trialkylaminoaryle, alcoxyC₁-C₃ ou hétérocyclique ; un radical arylC₆-C₁₈, éventuellement substitué par un ou plusieurs radicaux amino, hydroxy, alcoxy(C₁-C₃) ou alkyl(C₁-C₆) ; un radical carboxyalkyl(C₁-C₆) ou un radical alkylthio(C₁-C₆) ; un radical alcényle en C₂-C₆ ; ou
- R₁ et R₂, ensemble, forment avec l'atome d'azote auquel ils sont rattachés un hétérocycle ayant de 3 à 18 chaînons, pouvant contenir un ou plusieurs hétéroatomes supplémentaires choisis parmi un atome d'oxygène, d'azote ou de soufre, cet hétérocycle pouvant être substitué par un ou plusieurs radicaux alkyle en C₁-C₆, amino, acylamino C2-C6 ou hydroxyalkyle(C₁-C₄), le cycle ne comportant pas de liaison peroxyde, ni de radicaux diazo ou nitroso,
- R₃ représente un radical alkyle de 1 à 6 atomes de carbone, éventuellement substitué par un ou plusieurs atomes d'halogène, un ou plusieurs radicaux hydroxy, amino, aryle, alcoxyC₁-C₃ ; un radical arylC₆-C₁₈, éventuellement substitué par un ou plusieurs radicaux amino, hydroxy, alcoxy(C₁-C₃), alkyl(C₁-C₆) ; un radical carboxyalkyl(C₁-C₆) ou un radical alkylthio(C₁-C₆) ;
• au moins une base d'oxydation hétérocyclique à motifs pyrazoliques non condensés ;
• étant entendu que lorsque le coupleur pyridinique de formule (I) est choisi parmi la 2-(β-hydroxyéthylamino) 6-méthoxy 3-amino pyridine et la 3-amino 6-méthoxy 2-(méthylamino) pyridine, le pH de la composition prête à l'emploi est inférieur à 6,5.

2. Composition selon la revendication 1 dans laquelle R₂ représente un radical mono ou polyhydroxyalkyle en C₁-C₄.

3. Composition selon la revendication 1 dans laquelle R₁ et R₂ forment ensemble avec l'atome d'azote auquel ils sont attachés un cycle pyrrolidine, pipéridine, homopipéridine, imidazoline, pyrazolidine, piperazine.

4. Composition selon l'une quelconque des revendications 1 à 3 dans laquelle R₃ représente un radical alkyle en C1-C6, ou trifluoroalkyle.

5. Composition selon l'une quelconque des revendications 1 à 4 dans laquelle R4 est l'hydrogène.

6. Composition selon l'une quelconque des revendications 1 à 5 dans laquelle le coupleur pyridinique est choisi parmi le 6-méthoxy 3-amino 2-phénylaminopyridine, le 6-méthoxy 3-amino 2-(4'-hydroxyphényl)pyridine, 6-méthoxy 3-amino 2-(2'-méthoxyphényl)aminopyridine, 6-méthoxy 3-amino 2-(2'-hydroxyphényl)aminopyridine, 6-méthoxy 3-amino 2-diéthylaminopyridine, 6-méthoxy 3-amino 2-diméthylaminopyridine, le 6 méthoxy 3-amino 2-(méthyl, 2'-hydroxyéthyl)aminopyridine, 6-méthoxy 3-amino 2-(n-butyl, 2'hydroxyéthyl)pyridine, 6-méthoxy 3-amino 2bis-(2'hydroxyéthyl)aminopyridine, 6-méthoxy 3-amino 2-(2'3'dihydroxypropyl)aminopyridine, 6-méthoxy 3-amino 2-(1',1'-diméthyl 2'-hydroxyéthyl)aminopyridine, 6-méthoxy 3-amino 2-(1'-hydroxyméthyl 2'-hydroxyéthyl)aminopyridine, 6-méthoxy 3-amino 2-(1'-méthyl 2'-hydroxyéthyl)aminopyridine, 6-méthoxy 3-amino 2-(2'-hydroxypropyl)aminopyridine, 6-méthoxy 3-amino 2-(3'-diméthylaminopropyl)aminopyridine, 6-méthoxy 3-amino 2bis-(méthoxyéthyl)aminopyridine, 6-méthoxy 3-amino 2bis-(2'-propényl)aminopyridine, 6-méthoxy 3-amino 2-pyrrolidinylpyridine, 6-méthoxy 3-amino 2-(3'acétamidopyrrolidinyl)pyridine, 6-méthoxy 3-amino 2-(2'5'diméthylpyrrolidinyl)pyridine, 6-méthoxy 3-amino 2-(2'-diméthylaminoéthyl)aminopyridine, 6-méthoxy 3-amino 2-morpholinopyridine, 6-méthoxy 3-amino 2-(2'-méthylpyrrolidinyl)pyridine, 6-méthoxy 3-amino 2-pipérazinylpyridine, 6-méthoxy 3-amino 2-pyridinylpyridine, 6-méthoxy 3-amino 2-pyrrolidinylpyridine, 6-méthoxy 3-amino 2-(2'-méthylpyridinyl)pyridine, 6-méthoxy 3-amino 2-(2'-hydroxyéthylpyridinyl)pyridine, 6-méthoxy 3-amino 2-(2'-pyrrolidinyléthyl)aminopyridine, 6-méthoxy 3-amino 2-(3'imidazolinylpropyl)aminopyridine, 6-méthoxy 3-amino 2-[3'-(3"-méthylimidazolium)propyl)aminopyridine, 6-(2'-trifluoroéthoxy) 5-trifluorométhyl 2,3-diamino pyridine, 6-phénoxy 5-trifluorométhyl 2,3-diaminopyridine.

7. Composition selon la revendication 6 dans laquelle le coupleur pyridinique est choisi parmi le 6-méthoxy 3-amino 2-(2'3'-dihydroxypropyl)aminopyridine, 6-méthoxy 3-amino 2-(1'-méthyl 2'-hydroxyéthyl)aminopyridine, 6-méthoxy 3-amino 2-pyrrolidinylpyridine, 6-méthoxy 3-amino 2-(2'-méthylpyrrolidinyl)pyridine, 6-méthoxy 3-amino 2-(2'-méthylpyridinyl)pyridine, 6-méthoxy 3-amino 2-(2'-hydroxyéthylpyridinyl)pyridine.

8. Composition selon l'une quelconque des revendications 1 à 7 dans laquelle une base d'oxydation hétérocyclique à motifs pyrazoliques non condensés est une base d'oxydation diaminopyrazole de formule (III) 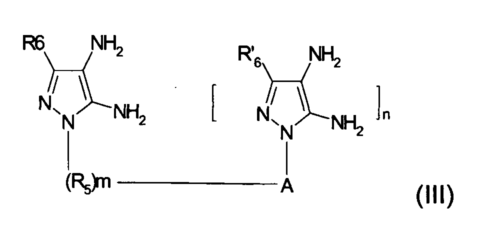 dans laquelle
• R₅, R₆ et R'₆ représentent indépendamment l'un de l'autre un atome d'hydrogène ; une chaîne alkyle de 1 à 6 atomes de carbone, éventuellement substituée par un ou plusieurs radicaux choisis parmi les radicaux hydroxy, amino, mono-, di- ou tri-alkylC1-C4amino, alcoxy(C₁-C₄), aryl(C₆-C₁₈) ou un ou plusieurs atomes d'halogène ; un radical aryle en C₆-C₁₈, éventuellement substitué par un ou plusieurs radicaux choisis parmi un radical amino, hydroxy, alcoxy(C₁-C₃), alkyle(C₁-C₆) ; un radical carboxyalkyl(C₁-C₆) ou alkylthio(C₁-C₆),
• n désigne 0 ou 1,
• m désigne 0 ou 1 à la condition que si n = 1 alors m = 0 et si n = 0 alors m= 1 ;
• A désigne une chaîne hydrocarbonée en C₁-C₂₀, linéaire ou ramifiée, cyclique ou non, saturée ou insaturée, hétérocyclique ou non, éventuellement interrompue par un ou plusieurs hétéroatomes, et éventuellement substituée par un radical hydroxy ou amino, un atome d'halogène.

9. Composition selon la revendication 8 dans laquelle n est égal à 0.

10. Composition selon la revendication 8 ou 9 dans laquelle R6 est un atome d'hydrogène ou un radical alkyle en C1-C4.

11. Composition selon la revendication 8 à 10 dans laquelle la base diaminopyrazole est choisie parmi le 4,5-diamino-1-β-hydroxyéthylpyrazole, le 4,5-diamino-1-éthyl 3-méthyl pyrazole et le 4,5-diamino 1-méthoxyéthylpyrazole

12. Composition selon la revendication 8 dans laquelle la base d'oxydation hétérocyclique à motifs pyrazoliques non condensés est un dimère de diaminopyrazole choisi parmi le 1,2 bis-[4,5-diamino-pyrazol-1-yl)éthane, le 1,4-bis-[4,5-diamino-pyrazol-1-yl méthyl]benzène, le 1,3-bis-(4,5-diamino-pyrazol-1-yl-méthyl)benzène, le 1,3-bis-(4,5-diamino-pyrazol-1-yl)propane.

13. Composition selon l'une quelconque des revendications précédentes comprenant une base d'oxydation additionnelle choisie parmi les para-phénylènediamines, les bis-phénylalkylènediamines, les para-aminophénols, les ortho-aminophénols, les bases hétérocycliques autre que les bases d'oxydation de formule (II) et leurs sels d'addition.

14. Composition selon l'une quelconque des revendications précédentes comprenant un coupleur choisi parmi les méta-phénylènediamines, les méta-aminophénols, les méta-diphénols, les coupleurs naphtaléniques, les coupleurs hétérocycliques autres que les coupleurs de formule (I) et leurs sels d'addition.

15. Composition selon l'une quelconque des revendications précédentes dans laquelle la quantité de chacune des bases d'oxydation est comprise entre 0,001 à 10 % en poids environ du poids total de la composition tinctoriale.

16. Composition selon l'une quelconque des revendications précédentes dans laquelle la quantité de chacun des coupleurs est comprise entre 0,001 et 10 % en poids environ du poids total de la composition tinctoriale.

17. Composition selon l'une quelconque des revendications 1 à 16 contenant de plus un agent oxydant.

18. Composition selon l'une quelconque des revendications 1 à 16 dans laquelle le pH est compris entre 3 et 12, de préférence entre 5 et 8.

19. Procédé de teinture d'oxydation des fibres kératiniques, **caractérisé en ce qu'**on applique sur les fibres une composition tinctoriale telle que définie dans l'une quelconque des revendications 1 à 16 en présence d'un agent oxydant pendant un temps suffisant pour développer la couleur désirée.

20. Procédé selon la revendication 19 dans lequel le pH de la composition prête à l'emploi est inférieur à 9.

21. Procédé selon la revendication 20 dans lequel le pH est inférieur ou égal à 7.

22. Procédé selon l'une quelconque des revendications 19 à 21 dans lequel l'agent oxydant est l'oxygène de l'air ou un agent choisi parmi le peroxyde d'hydrogène, le peroxyde d'urée, les bromates de métaux alcalins, les persels, les peracides et les enzymes oxydases.

23. Dispositif à plusieurs compartiments dans lequel un premier compartiment contient une composition tinctoriale telle que définie à l'une quelconque des revendications 1 à 16 et un deuxième compartiment contient un agent oxydant.

24. Kit pour la coloration des cheveux comprenant d'une part une composition tinctoriale telle que défini à la revendication 1 à 16 et d'autre part une composition contenant un agent oxydant.

25. Utilisation de la composition définie aux revendications 1 à 18 pour la teinture de fibres kératiniques.

26. Utilisation selon la revendication 25 pour la teinture des fibres kératiniques dans des nuances grise à noires.
